# EUROPEAN PATENT SPECIFICATION

(11) **EP 2 815 440 B1**
(45) Date of publication and mention of the grant of the patent: **11.07.2018**
(21) Application number: 13753213.1
(22) Date of filing: 12.02.2013
(51) Int. Cl.: H01L 35/32, H01L 35/34, A61F 7/00, H01L 21/04, A61F 7/02

(54) **MEMBRANE-SUPPORTED, THERMOELECTRIC COMPOSITIONS**
MEMBRANUNTERSTÜTZTE THERMOELEKTRISCHE ZUSAMMENSETZUNGEN
COMPOSITIONS THERMOÉLECTRIQUES PORTÉES PAR UNE MEMBRANE

(30) Priority: 16.02.2012 US 201213398567
(43) Date of publication of application: 24.12.2014
(73) Proprietor: Nanohmics, Inc., Austin, TX 78741 (US)
(72) Inventor: SAVOY, Steve, M., Austin, Texas 78736 (US); ZOLLARS, Byron, G., Georgetown, Texas 78628 (US); XUE, Qizhen, Austin, Texas 78750 (US); JOHN, Jeremy, J., Austin, Texas 78737 (US); PARKS, Paul, A., Austin, Texas 78704 (US)
(74) Representative: Jöstingmeier, Martin
(86) International application number: PCT/US2013/025785
(87) International publication number: WO 2013/165514

(56) References cited:
- US-A1- 2008 121 263
- US-B1- 6 872 879

## Description

### BACKGROUND OF THE INVENTION

### I. FIELD OF THE INVENTION

The present invention relates to the field of thermoelectric devices. In particular, thermoelectric compositions and devices formed with a porous membrane matrix, and methods for making and using such compositions and devices are described.

### II. BACKGROUND

The thermoelectric effect is the direct conversion between temperature difference and electric potential or vice versa. ThermoElectric Generators (TEGs), which operate under the principles of the Seebeck effect, generate an electric current from temperature differences, and conversely, ThermoElectric Coolers (TECs), which operate under the principles of the Peltier effect, generate a temperature difference with applied electric current. TEC and TEG devices are commercially available and are generally composed of alternating n-type and p-type semiconductor material referred to as ThermoElectric Elements (TEEs). Commercial TEEs can be composed of thin film, epitaxial layers (e.g., Nextreme, Durham, NC, USA) or bulk materials (*e.g.*, Marlow Industries, Dallas, TX, USA; Ferrotec, Santa Clara, CA, USA among others) such as extruded ingots that are cut to size and mechanically assembled on rigid ceramic substrates to form ThermoElectric Modules (TEMs).

Conventional methods for manufacturing bulk TEEs have included melt extrusion in the form of single and polycrystalline phases followed by mechanical processing to the desired shape of the TEE prior to placement in a TEM. Alternative approaches include vacuum deposition such as sputtering, electroplating, electrochemical and other slurry packing and compaction methods followed by sintering powdered material at high temperature and high pressure (i.e., hot pressing) (*e.g.*, U.S. Pat. No. 6,127,619 and U.S. Pat. Appl. Pub. No. 2008/0274004). Sintering processes vary, and-modifications may include the use of high-powered lasers (selective laser sintering), electricity (spark plasma sintering), or other mechanical processes such as embossing. These methods rely on deposition into a cavity mold to support the material during densification and consolidation in the formation of TEEs.

TEEs formed from nanometer-sized powders that are hot pressed into a bulk solid have been demonstrated to have higher thermoelectric performance compared to TEEs formed from larger sized powders. As a consequence, methods that provide a means to sinter nanometer-sized thermoelectric materials into bulk TEEs would enable fabrication of TEC/TEG devices with enhanced performance, as determined by a higher value of the thermoelectric figure of merit (Z), a dimensionless constant that describes the intrinsic thermoelectric property of a material, and is defined as the product of the Seebeck coefficient squared and the electrical conductivity divided by the thermal conductivity, σS²/κ. High-Z thermoelectric materials, in combination with efficient electrical and temperature control systems, are used to fabricate thermoelectric devices with large coefficients of performance (CoP), which is a metric for thermoelectric performance at the device level.

Most commercial TEC/TEG devices consist of mechanically assembled TEMs composed of semiconductor TEEs that are cuboid, arrayed, and capped on both ends with a rigid ceramic plate as the substrate that bears a patterned serpentine electrode for application or collection of the electric current. The TEM is mechanically assembled into an arrangement of TEEs with alternating polarity (*i.e.*, p-type and n-type) that are coated with a low contact resistance layer and bonded to electrodes. A thermoelectric device that is less rigid or even flexible would enable numerous applications in waste heat recovery and conformable device cooling that are not easily made compatible using the conventional rigid TEEs, TEMs and TEC/TEG devices.

Solid-state thermoelectric devices used for direct electrical to thermal energy conversion have been employed in various commercial settings such as cooling of detectors, computer chips and other consumer products including beverage cooling and automotive car seat cooling (Amerigon, Northville, MI, USA). These devices are constructed on rigid substrates and must be tiled to create large area structures.

A need exists for a thermoelectric device with high flexibility or conformability and a high coefficient of performance that can be readily manufactured using high throughput methods for both TEC and TEG devices. The device should ideally be adaptable for use in thermal contact with materials and surfaces having complex shapes, such as for: body-worn cooling; vehicular waste heat recovery; spacecraft applications; and lightweight, low density "open mesh" thermal interface and thermal boundary structures. Low density webs or large area sheets can also provide "breathability" for efficient convective air transport in, for example, body contact applications. Devices that can provide these properties and can be fabricated using sintering methods which yield TEEs with the enhanced thermoelectric properties observed with nanopowder materials do not exist in the art.

US20080121263A1 suggests manufacturing thermoelectric elements starting from a porous matrix or a porous substrate. The matrix consists of an electrically insulating material having sufficient thermal and chemical resistance as well as a low thermal conductivity. The matrix is provided in predetermined regions with different thermoelectric materials, so that continuous conductors are formed in the matrix. These are electrically connected to one another to form thermocouples, which in turn are electrically interconnected with one another to form the thermoelectric element.

### SUMMARY OF THE INVENTION

A method for making a membrane-supported thermoelectric subassembly is given by claim 1. A method for making a membrane-supported thermoelectric module is given by claim 6. Thermoelectric devices are disclosed having sintered thermoelectric powders in electrically isolated columns in a porous membrane. The isolated columns are electrically connected by patterned conducting layers on the membrane or on electrode-patterned thermal interface sheets registered and bonded to the membrane. The devices are made by dispensing thermoelectric powders in a membrane and sintering the powders with high-power pulsed irradiance. The devices may be used in applications benefiting from the flexibility of the membrane.

### BRIEF DESCRIPTION OF THE DRAWINGS

The following drawings form part of the present specification and are included to further demonstrate certain aspects of the invention. The invention may be better understood by reference to one or more of these drawings in combination with the detailed description of specific embodiments presented herein.
FIG. 1 is a flow chart diagram depicting the method for fabricating membrane-supported thermoelectric compositions including subassemblies, modules, and devices.
FIG. 2A-2D shows a cross section view of the process for dispersing thermoelectric powder, slurry or ink into membranes matrices and optical sintering to form consolidated thermoelectric elements.
FIG. 3A-3B is a cross section diagram illustrating complete fabrication of membrane-supported thermoelectric modules using roll-to-roll processing. FIG. 3A illustrates the process of fabricating the membrane-supported thermoelectric subassembly, and FIG. 3B illustrates the process of bonding the electrode-patterned thermal interface sheets to form the membrane-supported thermoelectric module.
FIG. 4 shows a cross section diagram illustrating one embodiment for registering and stacking multiple membrane-supported thermoelectric subassemblies to enhance the thermoelectric conversion efficiency.
FIG. 5 shows a cross section diagram illustrating one embodiment for stacking multiple membrane-supported thermoelectric modules into a thermoelectric device to enhance the cooling or power generation efficiency.
FIG. 6A-D are schematic representations illustrating the stages of making membrane-supported thermoelectric module comprising a single membrane-supported thermoelectric subassembly and two different types of consolidated thermoelectric elements.
FIG. 7 is a series of photomicrographs illustrating consolidated thermoelectric elements that have been consolidated by optical or photonic sintering and are dispersed across a membrane.

### DETAILED DESCRIPTION OF THE INVENTION

One embodiment of the invention is directed to membranous thermoelectric devices made with a porous membrane matrix and having thermoelectric materials dispersed within. As used herein, "membrane" refers to a thin, pliable sheet comprising a material, or composite of materials, that forms a porous matrix. As used herein, "thermoelectric membrane" or "membranous thermoelectric device" or "membrane-supported thermoelectric composition" or various combination of these terms refers to a membrane having two faces and comprising thermoelectric materials contained within an electrically insulating porous matrix. "Sheet" is used herein to refer to a flat composition that is thin relative to its length and width and is used interchangably with "strip", "ribbon", or "film" for referring to a sheet having any length or width. The lateral dimensions of a membrane can range from hundreds of micrometers up to tens of meters in width depending on the intended application.

Another embodiment of the present invention describes methods for making thermoelectric devices. Methods include delivering thermoelectric powders in the form of a dry powder or slurry or ink and optical or photonic, sintering of the dispersed powder to form consolidated thermoelectric elements. The terms "slurry", "slurries", "ink", and "inks" are used interchangeably herein and refer to thermoelectric powder mixed with or suspended in a liquid carrier. "Optical" or "photonic" sintering as used herein refers to the use of optical radiation to generate and apply heat to the slurries or powders to effect sintering or fusing. Optical or photonic sintering (as disclosed in U.S. Pat. No. 7,820,097, for example) is used for metal ink consolidation in flexible, printed electronics. The method involves delivering optical irradiance with high optical power density over a wide lateral area, but within a thin thickness range. Peak pulse energies delivered in such a fashion can result in local reflow of the material, yet thermal dissipation is sufficiently rapid that adjacent material does not heat up extensively or experience damage. Particle size of the thermoelectric powders is preferably in the range of nanoparticles. Nanoparticles refer to particles having dimensions less than about 1 micron. Generally, the largest particle dimension is less than about 500 nm, and it may be less than 100 nm.

In the present invention, optical sintering is used to consolidate thermoelectric powder into thermoelectric elements dispersed within and across the porous matrix of a membrane. As used herein the terms "matrix material", "membrane matrix", "matrix fibers", and "fibers" are used interchangeably and refer to the structural or skeletal material that makes up the non-porous component of a porous matrix. Matrix material or fibers used in the invention are frequently filamentous in nature, but may also be any shape or conformation that provides substance or texture, including but not limited to, spherical, cubical, rectangular, other geometrical shapes, or randomly or irregularly shaped. The skeletal material is usually a solid, but structures like low solid-volume-fraction materials such as foams may also be considered as useful in methods of the invention.

Initially, thermoelectric powder or a slurry/ink made by diluting the powder in a carrier fluid, is deposited onto a porous membrane matrix. Thermoelectric powder, slurry, or ink can be configured to span the entire thickness of the membrane, which can be from about 1 µm up to about 1 mm. In addition to serving as a mechanical support structure for the dispersed thermoelectric powder, the matrix material acts as a collection matrix during optical sintering when rapid heating induces reflow of thermoelectric powders. Reflow provides a means for thermoelectric particles to collect (*i.e.,* increase in density) along the surfaces of the matrix material selected for favorable surface interactions. Surface wetting properties of the matrix material can also dictate the *in situ* formation of nanostructured thermoelectric grains along the matrix material during energy dissipation (cooling). The formation of nanostructured regions may serve to enhance the thermoelectric figure of merit of the sintered bulk materials, which retain the beneficial properties of quantum confinement and high interface phonon scattering. The formation of these structures is the result of the interplay between surface tension and cohesive/adhesive forces of both the matrix material and the thermoelectric material. While not being bound by these remarks, it is believed that the thermoelectric material particles are partially liquefied and then solidify, as in a sintering process, but at least some particles may be totally melted and then re-solidify upon cooling after being influenced by the wetting properties of the matrix while in liquid form. Ultimately, the material structure and dimensions can be tailored by selection of specific wetting properties of materials comprising the membrane matrix and the irradiance flux on the system. The membrane matrix material may comprise various materials and material classes including glass/ceramic fibers, synthesized polymers (*e.g.*, polyester, polypropylene, nitrocellulose, etc.), natural fibers such as cotton and other glass/ceramic or glass/polymer composites such as fiberglass/aerogel blankets (e.g., Aspen Aerogels, Northborough, MA, USA) or fiberglass/polymer (*e.g.*, FUSION 5™, Whatman Inc., Piscataway, NJ, USA) for low thermal conductance across the porous matrix.

The selected thermoelectric material may comprise numerous different high figure-of-merit (*high-ZT*) thermoelectric materials such as bismuth/antimony tellurides, lead telluride for higher temperature operation, silver, copper and other transition metal tellurides, half-heusler compounds, and silicon/germanium for high temperature operation among other thermoelectric materials well-known in the art. The choice of thermoelectric material is unlimited provided that the material properties are advantageous for a given device setting and operational device temperature. In principle, any thermoelectric material, whether presently known in the art or that may be discovered in the future, can be employed in the present invention provided that a powder or a slurry/ink of the material can be made and that the material undergoes reflow and densification during sintering to form continuous bulk phase with sufficient electrical conductivity and an electrical percolation pathway that supports sufficient current densities for thermoelectric applications.

Fusion or sintering of crystalline domains of the thermoelectric material on the surface of the matrix material (*e.g.*, glass and other ceramics, polymers) provides a means to ensure consolidation of high thermoelectric figure of merit materials. With sufficient powder loading, high electrical transport across the membrane is achieved, and residual porosity of the matrix (*i.e.,* the open volume of the membrane matrix) is selected for a particular purpose. The degree of residual void space in the porous matrix, after consolidation of thermoelectric material, is dictated by the original volume of the thermoelectric powder, any pre-consolidation macroscopic densification processes such as external contact, and the number of consecutive applications/optical sintering processes invoked to fabricate the consolidated thermoelectric elements.

Ultimately, consolidated thermoelectric elements comprise a combination of thermoelectric material on the surface of the membrane matrix material and bulk interconnected thermoelectric material attached to the matrix fibers or present within pores or voids dispersed among the matrix. This composition collectively acts to carry current across the thermoelectric membrane. Bulk electrical transport across the membrane is mediated only through the consolidated thermoelectric elements in the membrane. Regions of the membrane matrix where consolidated thermoelectric elements are not formed (*i.e.,* where no thermoelectric powder is dispensed) are electrically insulating. Electrical isolation is ensured for neighboring thermoelectric elements not in contact with each other and across the thickness of the membrane (*i.e.,* opposite faces of the membrane). Therefore, the invention provides a means to create thermoelectric elements directly within a supporting porous matrix that limits electrical flow to only those regions with consolidated thermoelectric elements. The use of a low density matrix provides for low thermal conductance across the membrane, and thus, heat flow is also concentrated in the thermoelectric elements that have been consolidated within the membrane matrix. The ability to consolidate thermoelectric elements *in situ* within a supporting matrix provides a unique ability to simplify thermoelectric device production using high throughput techniques such as roll-to-roll manufacturing.

Optical sintering provides a means to consolidate all thermoelectric elements dispensed into a given footprint of a membrane. In one embodiment, limited, or no external pressure is applied to the membrane; however, pressure from a roller or dispensing head may be provided to assist bulk densification prior to sintering or fusing, and reflow of the thermoelectric materials (*i.e.*, combined contact pressure during dispensation). An apparatus comprising a dispenser, a means to apply contact pressure, and a means to impart optical sintering may be assembled for fabricating thermoelectric compositions of the present invention. This apparatus may include a transparent contact face that provides pressure while simultaneously providing a means to transmit light for optical sintering.

The porous matrix provides structural support for the membrane during dispensing/consolidation and is designed with a porosity that provides for wetting of the slurry or ink, densification of the powder, and adequate penetration of sintering light by virtue of guiding and scattering of the light through and off the membrane matrix material. The matrix material is also selected based on its ability to act as a standoff thermal insulating matrix that is flexible or conformable. In one aspect of the invention, it is desirable to use a membrane matrix that can be processed using high throughput roll-to-roll manufacturing methods. In other embodiments, it is not required that a membrane be amenable to roll-to-roll manufacturing methods and it may be fabricated in sheets. The matrix wettability can also serve as a means to prevent electrical material deposition such as during tinning of solder to specific individual thermoelectric elements formed in the membrane.

The porous nature of the thermoelectric membranes of the invention will enable new applications for lightweight, high surface area thermoelectric devices such as recovery of waste heat from pipes and vehicular components with large temperature differences from ambient. Other beneficial applications of the invention include human or animal body contact cooling; since the thermoelectric device will be more "breathable" for comfort and possibly include multiple subsystem circuits for staggering the cooling energy to different microdomains in contact with the body. This is particularly important for countering vasoconstriction-induced, heat flow restriction during active cooling of skin. Conformable thermoelectric bandages that can control heating and cooling for sports and other injuries, or induced hypothermia are also envisioned. For example, a thermoelectric device with a flexible maxtix as disclosed herein may be wrapped around a limb of a person, with a battery connected to the device. Activation of the device using the battery may heat or cool the area affected by the device. Cycling of heating and cooling may also be applied simply by reversing the polarity of the current applied.

Strips, ribbons, films or sheets of a membrane can be used for making a number of different types of conformable or flexible membrane-supported thermoelectric compositions of the invention. The registration and combination of various membrane-supported thermoelectric subassemblies of the invention with electrode-bearing thermal interface strips, ribbons, films or sheets provides a means for fabrication of various membrane-supported thermoelectric modules that can be further combined to fabricate conformable and flexible thermoelectric generator and cooler devices for numerous energy recovery and cooling applications.

Thermoelectric devices refer broadly to solid-state devices with assembled thermoelectric elements. In some embodiments, thermoelectric devices may be flexible or conformable to a surface on which the device may be applied. This is the case whether a device is being cooled directly or has a temperature differential that is used to generate an electric current in the device. In various aspects of the invention thermoelectric devices may be used to cool any surface that is part of any material. Some non-limiting examples of surfaces that may be cooled by thermoelectric cooling devices of the invention include parts of animal or human body tissue where comfort requirements dictate a certain device porosity or breathability that provides for circulation of ambient air, detectors for electromagnetic radiation or other diagnostic sensors, automotive car seats, beverage coolers or mattresses. In additional aspects of the invention, thermoelectric devices may be used for wrapping waste heat pipes, drains or other industrial process control devices. In still other aspects of the invention, thermoelectric devices of the invention may be used for generating energy such as for spacecraft and satellite energy generation. Furthermore, applications may include thermal energy scavenging in conjunction with other renewable energy collection such as photovoltaics, solar thermal, wind, nuclear, and isotopic decay.

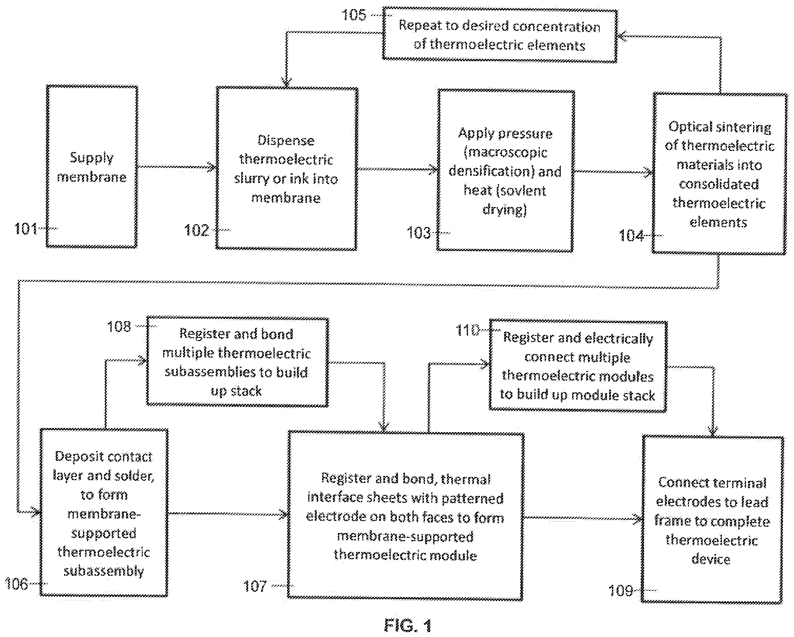

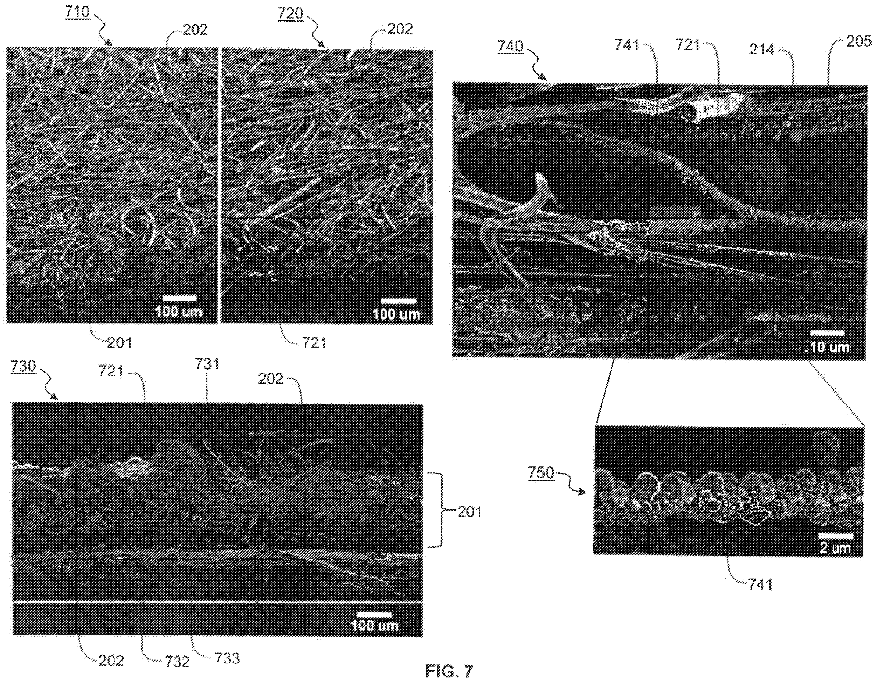

Referring to FIG. 1, methods for fabricating a membrane-supported thermoelectric device are illustrated. In this example, a membrane is supplied in step 101. Membranes of the invention also require sufficient electrical insulation so that electrical shorting does not occur between thermoelectric elements or across the membrane. Additionally, the membrane matrix should possess low intrinsic thermal conductivity to prevent thermal shorting across the membrane in regions remote from thermoelectric elements. A major consideration in membrane selection is the wettability of the surface of the matrix materials. The wetting properties can dictate the microcrystalline structure of the final consolidated thermoelectric element. Likewise, the surface wetting properties of matrix fibers dictate the degree of densification of the powder during fusion or sintering. This is described in more detail in FIG. 7.

The membrane matrix material should have a porosity that enables dispersion of thermoelectric powders or slurries within the matrix and across the full thickness of the membrane. The membrane matrix pore size, pore distribution, structure, and other physical and chemical properties, (such as for example, wetting by the slurry during dispensation of the thermoelectric powder with any densifier or binding agents) are important considerations for a given thermoelectric cooling or energy generation format. The morphology and the total thickness of the membrane also can influence sintering and consolidation of the thermoelectric powders and slurries into thermoelectric elements within the matrix. For example, the transparency of the matrix with respect to the irradiance spectrum will dictate the degree to which light can penetrate the membrane and be absorbed by thermoelectric powder embedded within the matrix. The relative solid volume fraction can also influence light scattering. For example, a higher density of matrix fibers can produce more scattering and affect the degree of light penetration into the membrane. Certain matrix fibers may act as guides for directing light into the matrix, where it can be absorbed by thermoelectric particles that are deeper in the matrix.

In step 102, thermoelectric powders or slurries are dispensed into selected areas of the membrane. Numerous methods may be used for dispensing thermoelectric dry powder and wet slurry or ink, including metered drop casting, aerosol jet, thermal spraying, and ink jet printing, to name a few. The lateral distribution and penetration depth of the thermoelectric material depend on the complex dynamics between the porosity of the membrane matrix material, surface wetting, and interaction with the solvent. In one embodiment, the matrix porosity and surface wetting characteristics are chosen such that the thermoelectric material is distributed across the entire thickness of the membrane such that electrical interfaces can be made on both faces of the membrane. This method greatly simplifies the application of electrode-bearing thermal interface sheets to each face of a membrane-supported thermoelectric subassembly. The lateral separation between dispensed thermoelectric materials and the composition of individual thermoelectric elements (e.g., n- and p-type) is variable and can be customized by using appropriate thermoelectric dispensing systems during manufacture. After dispensing the thermoelectric powders or slurries, additional macroscopic densification processes may be invoked, such as application of pressure and pre-baking to remove solvent, step 103. Contact pressure, alone or in conjunction with vibratory or ultrasonic action, may be employed during dispensing to move thermoelectric particles and matrix materials into closer contact and force particles to embed deeper in the membrane. In some embodiments, pressure and heat application, such as hot injection of the powder or slurry from a nozzle that locally compresses the membrane matrix, may occur along with the dispensing. An apparatus may also include the illumination element for optical sintering integrated with the dispensing/injection and contact pressure unit.

After dispensing the array of thermoelectric powders or slurries, sintering, step 104, is used to fuse the thermoelectric grains into a consolidated bulk thermoelectric material that forms a connected network within the membrane and is capable of conducting electricity. Although, as explained above, the powder may be fused or sintered or both. In one embodiment, optical, or photonic sintering is used to consolidate the thermoelectric material into thermoelectric elements. Other sintering methods such as hot pressing or spark plasma sintering may be used to consolidate the thermoelectric material into thermoelectric elements. The network of thermoelectric material generally follows the surfaces of the materials comprising the membrane matrix and in specific embodiments traverses the entire thickness of the membrane. The bulk material properties of electrical conductivity, thermal conductivity and Seebeck coefficient necessary to support high-ZT thermoelectric efficiency are generated during optical sintering within the membrane.

In some embodiments, the connected network is contiguous throughout the thermoelectric element and the matrix material serves only as a passive supporting matrix for the thermoelectric element. Additionally, the membrane thermal conductivity is kept low such that the membrane acts as a barrier or boundary to heat flow between the top and bottom faces, thus enhancing the efficiency of the thermoelectric composition. In some embodiments, membrane matrices such as aerogels or aerogel composite membranes (e.g., Aspen Aerogels, Northborough, MA, USA) are employed, and thermal conductivity is very low in the regions between consolidated thermoelectric elements. Additional material may be dispensed as needed to arrive at the final density and distribution of thermoelectric elements in the membrane, step 105.

After sintering, the exposed faces of the thermoelectric elements on each side of the membrane are conditioned with a low contact resistance material such as nickel and a solder film is added for making external electrical contacts. The membrane matrix material can act as a solder mask during deposition of the low contact resistance material and the solder. Alternatively, methods for deposition of the contact film and solder film include selective application on the consolidated thermoelectric elements using a printing technique or conversely using a mask to restrict application of the contact film and solder to the consolidated thermoelectric elements. In some aspects, each thermoelectric element receives a solder "bump" that can be bonded to a patterned electrode on the thermal interface sheets to form a thermoelectric module. The array of consolidated thermoelectric elements with electrical contact layers forms a thermoelectric subassembly, step 106.

The solder layer can then be used to bond the thermoelectric subassembly to thermal interface sheets on both faces to form a membrane-supported thermoelectric module, step 107. Thermal interface sheets have patterned electrodes that connect consolidated thermoelectric elements in a desired fashion to create a circuit. Alternatively, the electrode pattern may be deposited and patterned directly on the membrane-supported thermoelectric subassembly prior to bonding the thermal interface sheets. Prior to bonding thermal interface sheets, multiple membrane-supported thermoelectric subassemblies may be registered and stacked for purposes of improving thermoelectric efficiency, step 108. Typically, the thermal interface sheet matches the dimensions of the membrane and can take the form of thin strips, ribbons or sheets of thermal interface material. However, in some embodiments the thermal interface layer can take a different form than the membrane such as an array of thin strips aligned across the membrane width with fixed spacing, an array of small squares arranged in a checkerboard pattern or a singular sheet whose width is narrower than the membrane, to name a few examples. As a final measure, membrane-supported thermoelectric modules are cut to size and terminal contacts are added to form the membrane-supported thermoelectric device, step 109. Multiple thermoelectric modules may be electrically connected through bridging electrodes, which may also serve as mechanical connectors between modules in the formation of a module stack, step 110. Thermoelectric modules can be stacked to any useful and desired thicknesses and used in a variety of thermoelectric cooling and power generation device settings when the intermodule electrode contacts are made and the device is connected to an external circuit.

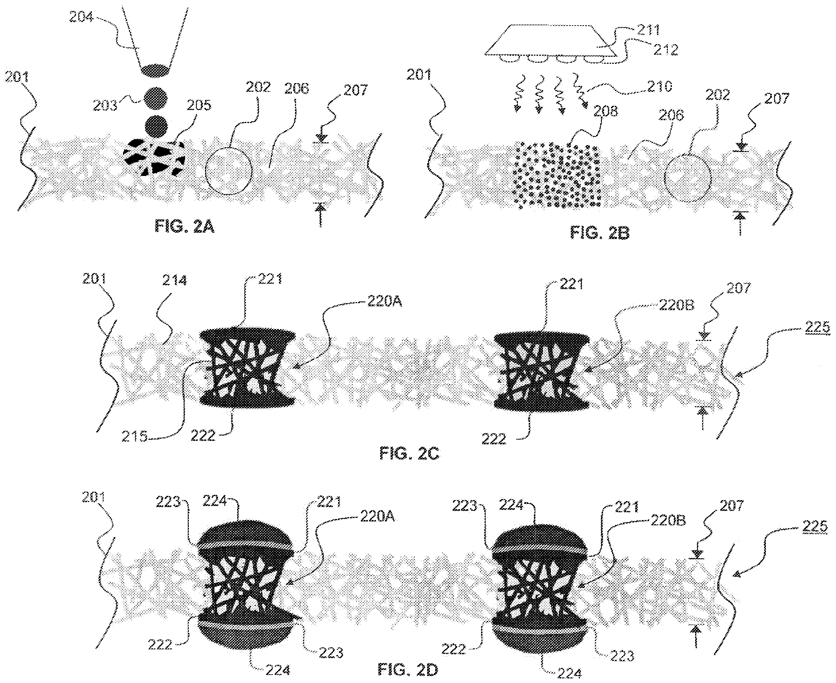

FIG. 2A illustrates a cross-sectional view of one embodiment of a method for making a membrane-supported thermoelectric subassembly 225. In certain embodiments of the invention, a membrane 201 may be any flexible or conformable material in the form of a thin film, sheet, or ribbon that is formed from a porous matrix (e.g., a polymeric membrane web, a thin film or roll of fiberglass, an aerogel fiberglass composite, or a fabric material to name a few). In other embodiments, flexibility and conformability are not necessary characteristics of the porous membrane matrix, and the membrane may be stiff, or even rigid. Such rigid, porous devices are useful in small area device applications such as detector cooling. Examples of stiff or rigid membranes include porous ceramics or glasses. Membrane 201 is supplied with a specific set of physical and chemical properties to support optical sintering of thermoelectric materials within porous membrane matrix 202. For the illustration in FIG. 2, membrane 201 is a material composition that is transparent to the optical radiation used for sintering, is electrically insulating and has a low thermal conductivity.

Appropriate amounts of thermoelectric powder, or a slurry or ink 203 composed of thermoelectric powder in a carrier fluid, is dispensed from dispensing unit 204 into specific areas of porous membrane matrix 202. Examples of thermoelectric powder would include materials from the families of bismuth or antimony tellurides, lead tellurides, silicon germanium, LAST compounds, half-heusler compounds and many other thermoelectric compounds with a range of doping levels to impart specific Seebeck coefficients, current carrying capacities and temperature range of operation to the final device. Thermoelectric powder from slurry or ink 203 disperses in porous membrane matrix 202 to form an unconsolidated column of thermoelectric powder 205 that fills some fraction of the void regions 206 of porous membrane matrix 202. Dispersion of thermoelectric powder from slurry or ink 203 can be controlled in both lateral directions to dictate the cross-sectional footprint of unconsolidated column of thermoelectric powder 205 and the vertical depth by adjusting the amount and duration of the application of thermoelectric powder from slurry or ink 203 from dispensing unit 204. Other factors affecting the distribution and penetration depth of thermoelectric powder from slurry or ink 203 into porous membrane matrix 202 include the application of heating, mechanical pressure, vibration, ultrasonication or combinations thereof during the dispensing step. In one embodiment, thermoelectric powder from slurry or ink 203 is dispersed across the entire thickness 207 of membrane 201.

Once thermoelectric powder from slurry or ink 203 is introduced into membrane 201, densification and consolidation of unconsolidated columns of thermoelectric powder 205 may be carried out, FIG. 2B, 2C. In one aspect of the invention, heating may be employed prior to consolidation to remove any carrier fluid and low molecular weight binder that may be present, yielding pre-densified thermoelectric powder element 208 that is dispersed in or across membrane 201, FIG. 2B. In another aspect of the invention, unconsolidated column of thermoelectric powder 205 may also be subjected to external pressure for powder compaction in addition to some level of heating, *i.e.,* "hot pressing" results in pre-consolidated thermoelectric powder element 208. This process can be time consuming and may affect the rate of the consolidation process which, in turn, affects the overall throughput in a roll-to-roll manufacturing process.

Optical sintering consists of exposing pre-densified thermoelectric powder element 208 with irradiance 210 using lamp assembly 211 which controls emission from lamps 212 such that pulsed power duration delivered to pre-densified thermoelectric powder element 208 leads to reflow and sintering without causing damage to membrane 201. In one embodiment, hot pressing is combined with optical sintering of pre-densified thermoelectric powder element 208. In another embodiment of the invention, optical sintering is used in the absence of heat or pressure to consolidate unconsolidated columns of thermoelectric powder 205. Furthermore, other methods, such as the application of electric current, may be employed solely or in combination with heat, pressure, or optical sintering to consolidate unconsolidated columns of thermoelectric powder 205. A suitable lamp assembly is available from Novacentrix (Austin, TX). It is a high-irradiance, pulsed source marketed as the PulseForge™ tool.

After sintering, pre-densified thermoelectric powder element 208 becomes consolidated thermoelectric element 220A as shown in FIG. 2C. In one embodiment, consolidation involves some degree of softening of pre-densified thermoelectric powder element 208. Alternately, full melting and reflow of liquid thermoelectric material (fusing) may occur during sintering of individual microcrystalline domains of pre-densified thermoelectric powder element 208. During reflow, the surfaces of matrix fibers 214 may facilitate wetting and hence assist in further densification of pre-densified thermoelectric powder element 208 by virtue of a change in the material phase from solid to liquid form. In this regard, "melted" pre-densified thermoelectric powder element 208 wets the surface of matrix fiber 214 and flows in such a fashion to coat the fiber surfaces 215, depicted as black fibers in FIG. 2C. During re-solidification when the local heating is dissipated, individual micro-/nanocrystalline domains can form as a result of the wetting of the matrix fibers 214 (not shown in FIG. 2C, and discussed further in FIG. 7).

Membrane 201 may comprise consolidated thermoelectric elements 220 having different compositions. For example, consolidated thermoelectric element 220A may have originated from a p-type thermoelectric powder while neighboring consolidated thermoelectric element 220B may have originated from an n-type thermoelectric powder. Both types of thermoelectric powders from slurry or ink 203 can be sintered from irradiance 210 in the same high throughput process.

In the embodiment depicted in FIG. 2C, consolidated thermoelectric elements 220 traverse the full thickness of membrane 201 such that a thermoelectric element top face 221 and thermoelectric element bottom face 222 are formed for each consolidated thermoelectric element 220. These faces serve as the electrical interface for each consolidated thermoelectric element 220 in porous membrane matrix 202. Consolidation of thermoelectric powders from slurry or ink 203 into one or more consolidated thermoelectric elements 220 across membrane 201 leads to the formation of a membrane-supported thermoelectric subassembly 225.

FIG. 2D illustrates the final step of adding electrical contact layer 223 followed by solder 224 to each thermoelectric element face 221 and 222 of all consolidated thermoelectric elements 220. Electrical contact layer 223 may consist of a low contact resistance material such as nickel or indium. Membrane-supported thermoelectric subassembly 225 can comprise a variety of configurations of thermoelectric elements 220, and the physical size (*e.g.,* diameter, square edge) and geometrical arrangement (*e.g.,* square or hexagonal packing) depends on the design chosen and on the manufacturing tools used to dispense the thermoelectric powder from slurry or ink 203.

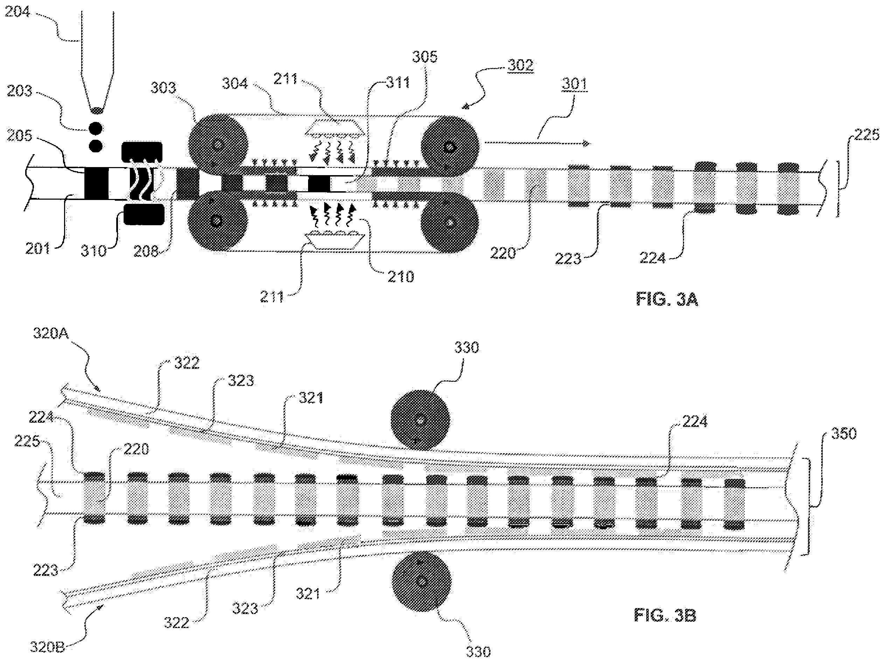

FIG. 3A illustrates one embodiment of an apparatus used in manufacturing membrane-supported thermoelectric subassemblies 225. The process begins with that described previously in FIG. 2. Thermoelectric powder from slurry or ink 203 is dispensed from dispensing unit 204 onto selected regions of membrane 201. As illustrated in FIG. 3A, membrane 201 is part of roll-to-roll system 302 wherein membrane 201 is being moved via rollers 303 through different processing regions in the direction illustrated by 301. In the embodiment illustrated in FIG. 3A, roll-to-roll system 302 has roller 303 and translation belt and belt frame 304, which can supply backside pressure 305 to guide membrane 201 through the sintering process. In another embodiment (not shown), force may be applied to nozzle 204 to press it against membrane 201 while thermoelectric powder from slurry or ink 203 are being dispensed through the nozzle. Additionally, lamp assembly 211 may be located near nozzle 204 such that optical sintering occurs during dispensing.

After dispensing thermoelectric powder from slurry or ink 203 into porous membrane matrix 202, heating, pressure, or some combination may occur in drying oven 310 for purposes of evaporating solvent and pre-densification of unconsolidated columns of thermoelectric powder 205. After traversing drying oven 310, pre-densified thermoelectric powder element 208 (FIG. 2) is formed. Membrane 201 bearing pre-densified thermoelectric powder element 208 is brought into optical sintering area 311 via roll-to-roll system 302. After exposure of pre-densified thermoelectric powder element 208 to irradiance 210 from lamp assembly 211, consolidated thermoelectric elements 220 are formed across membrane 201. Multiple lamp assemblies 211 may be used to sinter pre-densified thermoelectric powder element 208. Final processes for depositing electrical contact layer 223 and solder 224 onto consolidated thermoelectric element top face 221 (FIG. 2D) and bottom face 222 (FIG. 2D) lead to the formation of membrane-supported thermoelectric subassembly 225.

In one embodiment, membrane-supported thermoelectric subassembly 225 is ready to receive two terminal thermal interface films 320 as illustrated in FIG. 3B. When thermal interface films 320A and 320B are bonded to membrane-supported thermoelectric subassembly 225, membrane-supported thermoelectric module 350 is formed. Thermal interface films 320 serve as the outermost contact surface layer in a membrane-supported thermoelectric module 350 and are selected based on a combination of high thermal conductivity, ease of manufacturing and compatibility with an operational temperature range and the object to be contacted. Separate thermal interface films 320 are applied to the top 320A and bottom 320B of membrane-supported thermoelectric subassembly 225. In one embodiment, each thermal interface film 320 is attached to patterned electrode layer 321 and the pattern is different for the top film 320A and bottom film 320B to accommodate different electrical connectivity between consolidated thermoelectric elements 220 traversing membrane 201. In one embodiment, patterned electrode layer 321 is an electrical contact material disposed to electrically connect selected elements of thermoelectric material. On both consolidated thermoelectric element top face 221 and bottom face 222, patterned electrode layer 321 is designed to bridge individual, or selected groups of consolidated thermoelectric elements 220. Embodiments may include designs for a single electric circuit path, or alternatively multiple circuit paths that can be independently addressed. Registration of patterned electrode layer 321 is selected such that electrical connectivity is maintained between selected consolidated thermoelectric elements 220 using solder layer 224 as the bonding intermediary.

Thermal interface films 320 may comprise single and multiple thermal interface material layers. A bilayer embodiment of thermal interface film 320 is illustrated in FIG. 3B having a thicker thermal transport layer 322 (*e.g.,* a metal foil with high thermal conductivity such as copper or aluminum, noble metals, refractory metals and other non-metal or composites with high thermal conductivity) and a electrically insulating interface film 323 (*e.g.,* a polyimide film, for example Kapton®; E. I. du Pont de Nemours and Co., Wilmington, DE, USA). In some embodiments such as when ease and cost of manufacturing are considerations, thermal interface film 320 may comprise a polymer film alone wherein the overall thickness is kept low to keep the thermal conductivity high. Other embodiments may include ceramic sheets as the thermal interface films such as A1N or ceria. In some embodiments, electrically insulating interface film 323 may comprise a ceramic or glass layer such as silicon dioxide, silicon nitride or other oxide insulators that are deposited using vacuum deposition techniques such as sputtering, thermal evaporation, or e-beam evaporation. In other embodiments, a high thermal conductivity layer such as aluminum nitride (AlN) maybe be deposited as electrically insulating interface film 323. Electrically insulating interface film 323 serves to electrically isolate thermal transport layer 322 from patterned electrode layer 321 and provides low thermal resistance.

Electrical connectivity between consolidated thermoelectric elements 220 is made through heated roller pressure applicator 330, which induces the reflow of solder 224 and bonding to specific, registered locations on patterned electrode layer 321. Other non-limiting methods such as a reflow oven or sheet press (not shown in Fig. 3B) may be used to bond thermal interface sheets 320 to membrane-supported thermoelectric subassemblies 225. After bonding of thermal interface films 320 to membrane-supported thermoelectric subassembly 225, membrane-supported thermoelectric module 350 is formed. Membrane-supported thermoelectric module 350 serves as the primary thermoelectric composition that can be cut to size, aligned and stacked into different arrangements to form membrane-supported thermoelectric devices.

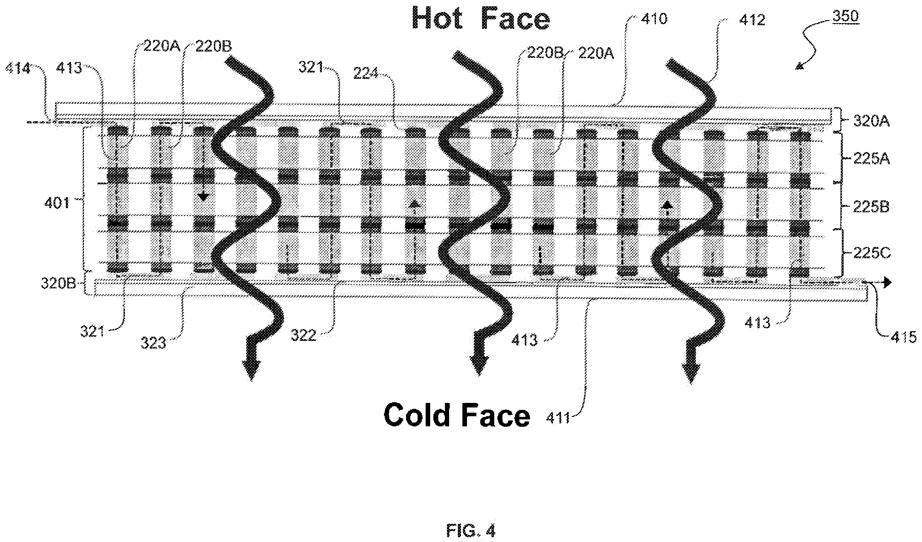

In one embodiment, methods are used to increase thermoelectric efficiency with registration and stacking of multiple membrane-supported thermoelectric subassemblies 225 prior to attachment of thermal interface film 320. FIG. 4 illustrates the embodiment having three thermoelectric subassemblies 225A-C registered and bonded to form a membrane-supported thermoelectric subassembly stack 401. In practice, however, any number (N) of membrane-supported thermoelectric subassemblies 225 may be used to make a membrane-supported thermoelectric subassembly stack 401. Stacking multiple membrane-supported thermoelectric subassemblies 225 in this fashion includes registering and bonding solder 224 from one consolidated thermoelectric element 220 directly to another consolidated thermoelectric element 220 in the adjacent membrane-supported thermoelectric subassembly 225. As a final measure, the two outside thermoelectric subassemblies 225 are bonded to thermal interface films 320 to form membrane-supported thermoelectric module 350. Either a single membrane-supported thermoelectric subassembly 225, or a membrane-supported thermoelectric subassembly stack 401 can form a membrane-supported thermoelectric module 350 when thermal interface films 320 are bonded to the outermost faces 221 and 222 of membrane-supported thermoelectric subassembly 225.

In the embodiment depicted in FIG. 4, membrane-supported thermoelectric subassembly stack 401, has a hot face 410 and cold face 411. In applications where heat flux 412 is imparted across thermoelectric module 350 (generator mode), an electric current 413 (dotted line) is generated. Electric current 413 flows across multiple thermoelectric elements 220 contained in membrane-supported thermoelectric subassemblies 225A-N. Electric current 413 is partially illustrated taking a serpentine path through individual consolidated thermoelectric elements 220 contained in membrane-supported thermoelectric subassembly stack 401 beginning with current input lead 414, traveling down through registered, consolidated thermoelectric elements 220A in membrane-supported thermoelectric subassemblies 225A-C, reversing in patterned electrode layer 321 and returning up the stack through registered, consolidated thermoelectric elements 220B. Electric current 413 continues through the full set of consolidated thermoelectric elements 220 although only part of the full current trajectory is illustrated in FIG. 4. At the right end of membrane-supported thermoelectric subassembly stack 401, electric current 413 exits at terminal lead 415. Conversely, the application of electric current 413 through membrane-supported thermoelectric subassembly stack 401 leads to the formation of heat flux 412 across, membrane-supported thermoelectric module 350 (cooler/heater mode).

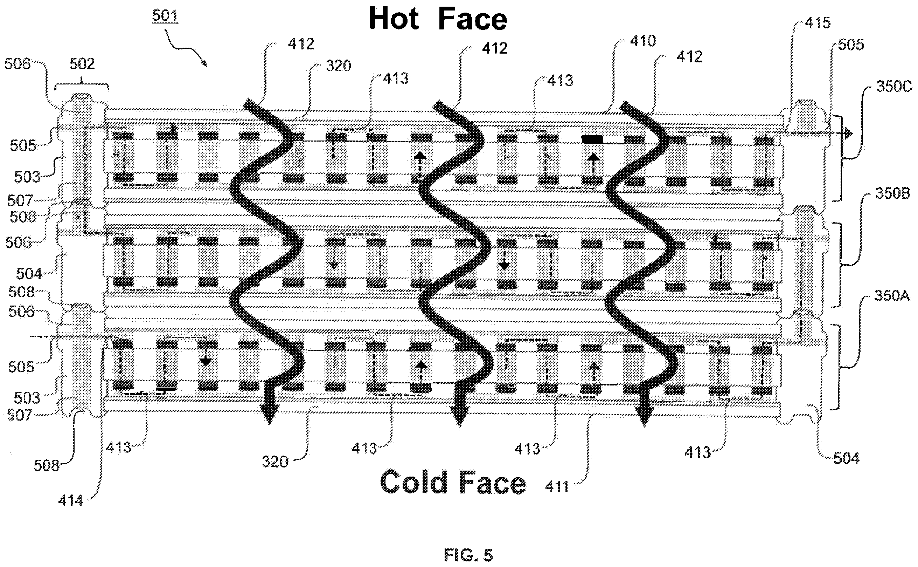

Any combination of membrane-supported thermoelectric modules 350 can be made to form thermoelectric device 501, FIG. 5. Thermoelectric device 501 may comprise membrane-supported thermoelectric modules 350 in any combination of membrane-supported thermoelectric subassemblies 225 or membrane-supported thermoelectric subassemblies stack 401. However, for illustrative purposes here and to assist in visualizing embodiments of the invention, only membrane-supported thermoelectric modules 350 comprising single layer membrane-supported thermoelectric subassemblies 225 are shown in FIG. 5. Microscale registration between thermoelectric elements 220 is not required when connecting membrane-supported thermoelectric modules 350 to form thermoelectric device 501 depicted in FIG. 5 since membrane-supported thermoelectric module 350 already bears thermal interface films 320 on membrane-supported thermoelectric subassemblies 225 (as depicted in FIG. 3B). Each membrane-supported thermoelectric module 350 may be designed with a specific size, for example, to increase the heat removing capacity of the device.

FIG. 5 illustrates thermoelectric device 501 having three membrane-supported thermoelectric modules 350A-C, stacked and electrically connected to form a contiguous serpentine electrical pathway for electric current 413. As illustrated in FIG. 4, electric current 413 originates at current input lead 414 and exits at terminal lead 415. For electrical interconnection between membrane-supported thermoelectric modules 350, edge connectors 502 are used. Each connector 502 can also provide mechanical support for thermoelectric device 501. Edge connectors 502 are designed to match the layer thickness of membrane-supported thermoelectric module 350 and not inhibit conformability of thermoelectric device 501. Edge connectors 502 are tethered to membrane-supported thermoelectric module 350 and are used to make protected electrical connections and mechanical anchoring of each membrane-supported thermoelectric module 350 in thermoelectric device 501. Means for interconnection between membrane-supported thermoelectric modules 350 include adhesives, snapping, and fastening using for example, edge connectors, to name a few non-limiting examples. Many embodiments are possible for each kind of electrical connection device. Snap together devices provide a certain degree of modularity for readily supplying power collection and heat transport capabilities of thermoelectric device 501. Edge connectors 502 may comprise two different types including feedthrough connectors 503 and directional connectors 504. Feedthrough connectors 503 have an edge terminal 505 that can be used to make connections to an external circuit (not shown). Additionally, feedthrough connectors 503 have a top terminal 506 and a bottom terminal 507, which can be used to make connections between individual membrane-supported thermoelectric modules 350 in thermoelectric device 501 via snap junction 508 between membrane-supported thermoelectric modules 350. Directional connectors 504 are used on the opposite edge of the same membrane-supported thermoelectric module 350B. Directional connectors 504 have both edge terminal 505 and top terminal 506, but lack bottom terminal 507 and make only mechanical connection through snap junction 508. The pattern is reversed on adjacent membrane-supported thermoelectric modules 350 and is designed to send electric current 413 through a serpentine path across all membrane-supported thermoelectric modules 350 in thermoelectric device 501. Only a fraction of the electrical current path is shown, for clarity, in FIG. 5.

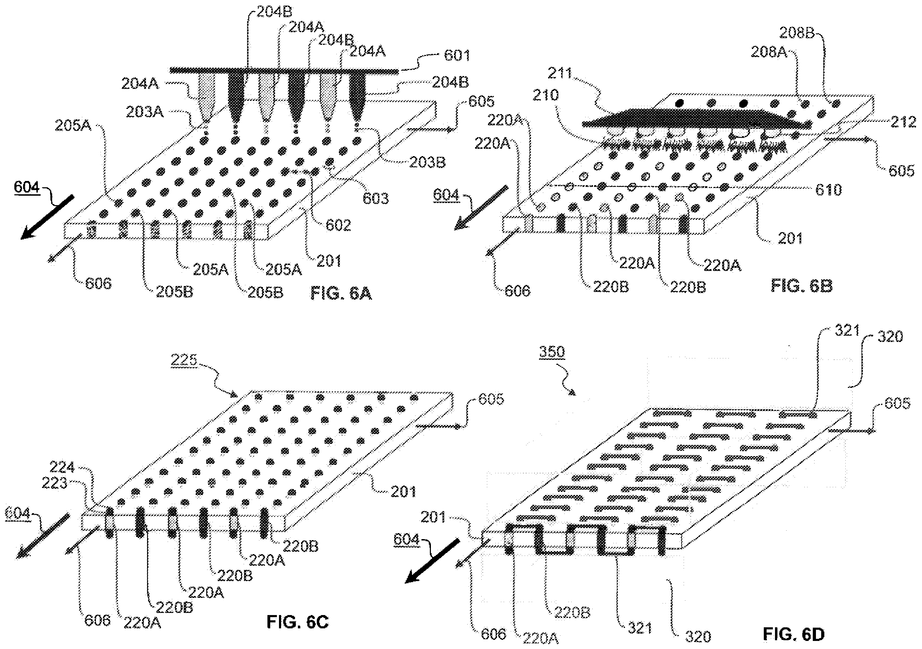

FIG. 6 illustrates a method for making one embodiment of the invention that includes a membrane-supported thermoelectric module 350 comprising a single membrane-supported thermoelectric subassembly 225 and two different types of consolidated thermoelectric elements 220A and 220B. In the geometry illustrated in FIG. 6A, consolidated thermoelectric element 220A is formed using thermoelectric powder from slurry or ink 203A and represents a p-type thermoelectric material, and 220B is formed using thermoelectric powder from slurry or ink 203B and represents an n-type thermoelectric material. In this embodiment, consolidated thermoelectric elements 220 are formed in locations where thermoelectric powder from slurry or ink 203 is dispensed into porous membrane matrix 202 from a linear array of dispensers 601. The illustration depicts alternating dispensing units for p-type 204A and n-type 204B used to dispense thermoelectric powder from slurry or ink 203A and 203B, respectively. In practice, any arrangement of dispensing units 204 for dispensing thermoelectric powder from slurry or ink 203 can be used provided that the arrangement enables facile electrical connection between consolidated thermoelectric elements 220. For example, the pitch 602 between unconsolidated columns of thermoelectric powder 205, the spot width 603, the packing density and the packing type such as linear, cubic, hexagonal are all defined by the arrangement of dispenser units 204 for membrane 201 that, in one embodiment of the invention, is part of roll-to-roll system 302 moving in the direction illustrated by 604. The width 605 and length 606 of membrane 201 can be any dimension that is suitable for manufacturability. Membranes 201 having larger membrane widths 605 can be cut into thinner strips for packaging smaller devices, and methods for cutting membranes into any length 606 can use, for example, converting manufacturing practices that are well-known in the art.

FIG. 6B illustrates optical sintering of pre-densified thermoelectric powder element 208 into consolidated thermoelectric elements 220 under irradiance 210 from lamp assembly 211. A separate process, not illustrated in Fig. 6, is used to create pre-densified thermoelectric powder elements 208 from unconsolidated columns of thermoelectric powder 205. This process was illustrated previously in FIG. 2B, 2C, and the cross section view illustrated in FIG. 2C is represented here by dashed line 610. All pre-densified thermoelectric powder elements 208 which are under irradiance 210 during a fixed exposure profile (*e.g.*, exposure duration, lamp 212 pulse rate, power density, etc.) form consolidated thermoelectric elements 220 during the traversal of membrane 201 through lamp housing 211 exposure region. In FIG. 6B, exposure is illustrated only on the top face 221 of membrane 201. In practice, exposure may occur on both faces 221 and 222 of membrane 201 at any angle that supports ease of consolidation. In the embodiment pictured in FIG. 6B, cross sections through membrane 201 represent a line of alternating p-type 220A and n-type 220B consolidated thermoelectric elements. Electrical contact layer 223 and solder 224 are then applied to all faces 221 and 222 of consolidated thermoelectric elements 220. Electrical contact layer 223 may be deposited using well-known methods such as electroless or vacuum deposition. Electroless deposition of solder 224 may be carried out using a reflow oven where membrane 201 may act as a wetting barrier to solder 224 analogous to solder masks used in the manufacture of printed circuit boards. FIG. 6C illustrates a completed membrane-supported thermoelectric subassembly 225 after electrical contact layer 223 and solder 224 have been deposited.

In a final step, thermal interface films 320 are bonded to membrane-supported thermoelectric subassembly 225 to form membrane-supported thermoelectric module 350 (FIG. 6D). The dimensions of thermal interface films 320 and patterned electrode layer 321, and any electrically insulating layers 322 (not seen in FIG. 6D) are selected to match width 605 and length 606 of membrane-supported thermoelectric subassembly 225. Thermoelectric device 501 can then be fabricated from thermoelectric modules 350 which can comprise one or more membrane-supported thermoelectric subassembly 225, one or more membrane-supported thermoelectric subassembly stack 401, or various combinations of membrane-supported subassembly or subassembly stacks.

FIG. 7 illustrates a series of SEM photomicrographs that depict the results of optical or photonic sintering of thermoelectric powder from slurry or ink 203 into consolidated thermoelectric elements 220. SEM photomicrograph 710 shows membrane 201 prior to addition of thermoelectric powder from slurry or ink 203 into porous membrane matrix 202. SEM photomicrograph 720 shows membrane 201 after thermoelectric powder from slurry or ink 203 has been injected and undergone optical or photonic sintering to form consolidated thermoelectric elements 220. In this example, membrane 201 comprises porous membrane matrix 202 having a blend of polymers and glass and is sold under the trademark name FUSION 5™ (Whatman Inc., Piscataway, NJ, USA). The FUSION 5 ™ membrane 201 is just one example of many possible matrix types that may be used in construction of thermoelectric devices 501 of the invention. This example illustrates the situation in which low volumetric fill of thermoelectric powder from slurry or ink 203 was introduced. Only the largest thermoelectric domains 721, formed during optical sintering, can be seen in the image compared to the bulk. In contrast, SEM photomicrograph 730 illustrates the situation in which a large volumetric fill of thermoelectric powder from slurry or ink 203 was introduced into porous membrane matrix 202. In this instance, a large degree of bulk material phase can be seen and matrix fibers 214 of membrane 201 are occluded and completely surrounded by thermoelectric domains 721 formed from thermoelectric powder from slurry or ink 203 in porous membrane matrix 202. The boundary 731, represented by the dotted line,between consolidated thermoelectric element 220 and a region of porous membrane matrix 202 where no thermoelectric powder from slurry or ink 203 was introduced can be clearly seen in the cross section image which depicts membrane 201 adhered to stage 732 by copper tape 733 for purposes of collecting SEM photomicrograph 730

Reflow and recrystallization dynamics can be influenced by the volume and amount of thermoelectric powder from slurry or ink 203 dispensed into porous membrane matrix 202. In SEM photomicrograph 740, the volume of thermoelectric powder from slurry or ink 203 is low relative to the total void fraction 206 of porous membrane matrix 202. In this embodiment, thermoelectric powder from slurry or ink 203 can reflow during optical sintering under irradiance 210. If porous membrane matrix 202 is selected with specific wetting characteristics, then the liquid formed during melting of thermoelectric powder from slurry or ink 203 will wet the surface and flow along matrix fibers 214 of porous membrane matrix 202 during irradiance 210. After dissipation of the heat, balance between the cohesive forces in the melted thermoelectric fluid and the adhesive, or wetting forces, on the matrix fibers 214 will dictate formation of consolidated thermoelectric element 225. In some instances, melting of thermoelectric powder from slurry or ink 203 withinporous membrane matrix 202 may lead to the formation of electrically-conducting, sintered micro- and nanophase thermoelectric domains 741. The formation of micro- and nanophase thermoelectric domains 741 may provide enhanced thermoelectric efficiency due to increased phonon scattering and quantum confinement effects. Inset SEM photomicrograph 750 illustrates a segment of a single matrix fiber 214 of porous membrane matrix 202 that is coated with electrically-conducting sintered micro- and nanophase thermoelectric 741 after sintering.

It is understood that modifications to the invention may be made as might occur to one skilled in the field of the invention within the scope of the appended claims. All embodiments contemplated hereunder which achieve the objects of the invention have not been shown in complete detail. Other embodiments may be developed without departing from the scope of the appended claims. Although the present invention has been described with respect to specific details, it is not intended that such details should be regarded as limitations on the scope of the invention, except to the extent that they are included in the accompanying claims.

## Claims

1. A method for making a membrane-supported thermoelectric subassembly (225), comprising:
providing a porous membrane (201) having a top face and a bottom face;
dispensing a thermoelectric powder or slurry of powder (203) into a plurality of selected areas in the top face or bottom face of the porous membrane so as to place the powder in electrically isolated columns (205) extending to the top face and the bottom face of the membrane; and
sintering the thermoelectric powder in the membrane to form a plurality of consolidated thermoelectric elements (220A),
**characterized in that**
sintering is caused by irradiance from a pulsed lamp assembly (211).

2. The method of claim 1 further comprising compressing the membrane (201).

3. The method of claim 2 wherein compressing the membrane (201) is caused by passage of the membrane between rollers (303).

4. The method of claim 2 wherein compressing the membrane (201) is caused by force of a nozzle (204) on the membrane (201).

5. The method of claim 1 further comprising placing solder (224) on the thermoelectric elements (220A).

6. A method for making a membrane-supported thermoelectric module (350), comprising:
providing a porous membrane (201) having a top face and a bottom face;
dispensing a thermoelectric powder or slurry of powder (203) into a plurality of selected areas in the top face or bottom face of the porous membrane (201) so as to place the powder in spaced-apart columns (205) extending to the top face and the bottom face of the membrane (201);
sintering the thermoelectric powder in the membrane (201) to form a plurality of consolidated thermoelectric elements (220A); and
applying a thermal interface film (320, 320A, 320B) to each of the top face and the bottom face of the membrane (201), each thermal interface (320, 320A, 320B) film having a patterned electrode layer (321) thereon,
**characterized in that**
sintering is caused by irradiance from a pulsed lamp assembly (211).

## Patentansprüche

1. Verfahren zur Herstellung einer membrangestützten thermoelektrischen Unteranordnung (225), umfassend:
Bereitstellen einer porösen Membran (201) mit einer oberen Fläche und einer unteren Fläche;
Abgeben eines thermoelektrischen Pulvers oder einer Pulversuspension (203) in eine Mehrzahl von ausgewählten Bereichen in der oberen Fläche oder unteren Fläche der porösen Membran, um das Pulver in elektrisch isolierte Säulen (205) anzuordnen, die sich zu der oberen Fläche und der unteren Fläche der Membran erstrecken; und
Sintern des thermoelektrischen Pulvers in der Membran, um eine Mehrzahl von konsolidierten thermoelektrischen Elementen (220A) zu bilden,
**dadurch gekennzeichnet, dass**
das Sintern durch Bestrahlen von einer gepulsten Lampenanordnung (211) verursacht wird.

2. Verfahren nach Anspruch 1, weiterhin umfassend das Komprimieren der Membran (201).

3. Verfahren nach Anspruch 2, wobei das Komprimieren der Membran (201) durch das Passieren der Membran zwischen Walzen (303) bewirkt wird.

4. Verfahren nach Anspruch 2, wobei das Komprimieren der Membran (201) durch Kraftausübung einer Düse (204) auf die Membran (201) bewirkt wird.

5. Verfahren nach Anspruch 1, weiterhin umfassend das Anordnen von Lötmittel (224) auf den thermoelektrischen Elementen (220A).

6. Verfahren zur Herstellung eines membrangestützten thermoelektrischen Moduls (350), umfassend
Bereitstellen einer porösen Membran (201) mit einer oberen Fläche und einer unteren Fläche;
Abgeben eines thermoelektrischen Pulvers oder einer Pulversuspension (203) in eine Mehrzahl von ausgewählten Bereichen in der oberen Fläche oder unteren Fläche der porösen Membran (201), um das Pulver in beabstandeten Säulen (205) anzuordnen, die sich zu der oberen Fläche und der unteren Fläche der Membran (201) erstrecken; Sintern des thermoelektrischen Pulvers in der Membran (201), um eine Mehrzahl von konsolidierten thermoelektrischen Elementen (220A) zu bilden; und
Aufbringen eines thermischen Grenzflächenfilms (320, 320A, 320B) auf jeweils die obere Fläche und die untere Fläche der Membran (201), wobei jeder thermische Grenzflächenfilm (320, 320A, 320B) eine strukturierte Elektrodenschicht (321) darauf aufweist,
**dadurch gekennzeichnet, dass**
das Sintern durch Bestrahlen von einer gepulsten Lampenanordnung (211) verursacht wird.

## Revendications

1. Procédé de fabrication d'un sous-ensemble thermoélectrique (225) supporté par une membrane, consistant à :
fournir une membrane poreuse (201) ayant une face supérieure et une face inférieure ;
distribuer une poudre thermoélectrique ou une suspension de poudre (203) dans une pluralité de zones sélectionnées dans la face supérieure ou la face inférieure de la membrane poreuse de façon à placer la poudre dans des colonnes électriquement isolées (205) s'étendant vers la face supérieure et la face inférieure de la membrane ; et
fritter la poudre thermoélectrique dans la membrane pour former une pluralité d'éléments thermoélectriques consolidés (220A),
**caractérisé en ce que**
le frittage est produit par l'irradiance d'un ensemble formant lampe pulsée (211).

2. Procédé selon la revendication 1, consistant en outre à comprimer la membrane (201).

3. Procédé selon la revendication 2, dans lequel la compression de la membrane (201) est produite par passage de la membrane entre des rouleaux (303).

4. Procédé selon la revendication 2, dans lequel la compression de la membrane (201) est produite par la force d'une buse (204) sur la membrane (201).

5. Procédé selon la revendication 1, consistant en outre à placer de la soudure (224) sur les éléments thermoélectriques (220A).

6. Procédé de fabrication d'un module thermoélectrique (350) supporté par une membrane, consistant à :
fournir une membrane poreuse (201) ayant une face supérieure et une face inférieure ;
distribuer une poudre thermoélectrique ou une suspension de poudre (203) dans une pluralité de zones sélectionnées dans la face supérieure ou la face inférieure de la membrane poreuse (201) de façon à placer la poudre dans des colonnes espacées (205) s'étendant vers la face supérieure et la face inférieure de la membrane (201) ;
fritter la poudre thermoélectrique dans la membrane (201) pour former une pluralité d'éléments thermoélectriques consolidés (220A) ; et
appliquer un film d'interface thermique (320, 320A, 320B) sur la face supérieure et sur la face inférieure de la membrane (201), chaque film d'interface thermique (320, 320A, 320B) étant recouvert d'une couche d'électrode structurée (321),
**caractérisé en ce que**
le frittage est produit par l'irradiance d'un ensemble formant lampe pulsée (211).
